# EUROPEAN PATENT SPECIFICATION

(11) **EP 1 699 753 B1**
(45) Date of publication and mention of the grant of the patent: **26.11.2014**
(21) Application number: 03780615.5
(22) Date of filing: 29.12.2003
(51) Int. Cl.: C07C 253/30, C07C 255/41

(54) **IMPROVED PROCESS FOR THE PREPARATION OF ENTACAPONE**
VERBESSERTES VERFAHREN ZUR HERSTELLUNG VON ENTACAPON
PROCEDE AMELIORE DE PREPARATION DE L'ENTACAPONE

(43) Date of publication of application: 13.09.2006
(73) Proprietor: Suven Life Sciences Limited, Hyderabad 500 034 (Andra Pradesh) (IN)
(72) Inventor: VEERA REDDY, A.; c/o SUVEN LIFE SCIENCES LTD, 500034 HYDERABAD (IN); RAJENDIRAN, C.; c/o SUVEN LIFE SCIENCES LTD, 500034 HYDERABAD (IN); MD QADEERUDDIN; c/o SUVEN LIFE SCIENCES LTD, 500034 HYDERABAD (IN); JASTI, Venkateswarlu; c/o SUVEN LIFE SCIENCES LTD, 500034 HYDERABAD (IN)
(74) Representative: Harris, Jennifer Lucy
(86) International application number: PCT/IN2003/000401
(87) International publication number: WO 2005/063693

(56) References cited:
- US-A- 4 963 590
- US-A- 5 981 569
- WIKBERG T ET AL: "Identification of Major Metabolites of the Catechol-O-Methyl Transferase Inhibitor Entacapone in Rats and Humans" DRUG METABOLISM AND DISPOSITION, WILLIAMS AND WILKINS., BALTIMORE, MD, US, vol. 21, no. 1, 1 January 1993 (1993-01-01), pages 81-92, XP009064899 ISSN: 0090-9556

## Description

The present invention relates to an improved process for the preparation of ENTACAPONE. ENTACAPONE having the formula 1 is a potent and specific peripheral catechol-O-methyltransferase (COMT) inhibitor. It is used in combination with levodopa / carbidopa to treat Parkinson's disease, sometime referred to as shaking palsy. Entacapone enhances the effect of levedopa / carbidopa by improving muscle control.

The invention also relates to a novel intermediate of the formula 4 where R =ethyl and a process for its preparation

The preparation of Entacapone of the formula (1) has been reported in GB 2200109 and US Patent No 4963590 (1987, 1990,Orion-Yhtymaeoy (FI)) from two critical intermediates viz; 3,4-dihydroxy-5-nitrobenzadehyde of the formula (5) and N,N-diethylaminocyanoacetamide of the formula (3). The compound of the formula 5 is condensed with the compound of the formula(3) in the presence of piperidine acetate and dry ethanoll as a solvent gave Entacapone of the formula (1). The 3,4-dihydroxy-5-nitrobenzaldehyde of the formula (5) in turn was prepared from 3-methoxy-4,hydroxy-5-nitrobenzaldehyde(6) using acetic acid and hydrobromic acid as described in scheme-1.

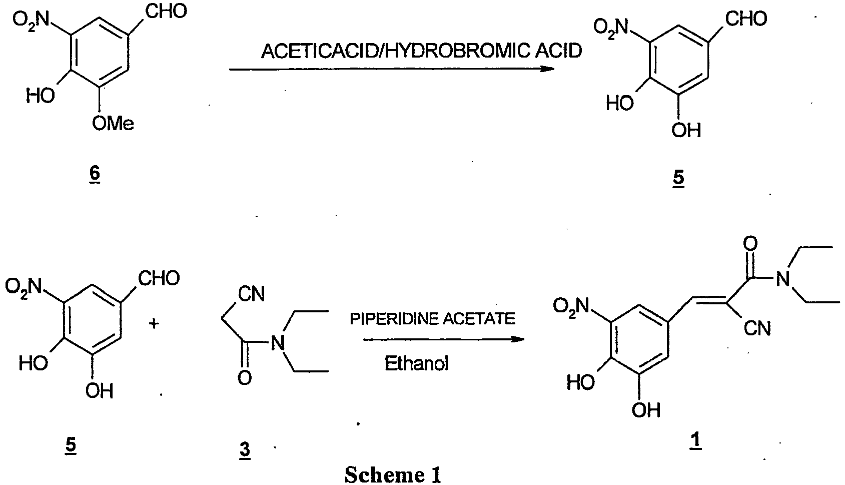

The above patent has described the preparation of the entacapone of the formula (1) without describing the stereochemistry or the polymorphism.

Subsequently it was described in the US Patent No 5135950 (1992, Orion Orion-Yhtymaeoy (FI)) about preparing E-isomer and polymorphism-A from the mixture abtained from the reaction reported in the GB patent No 2200109.

The main disadvantage of this method, according to our findings, is that the reaction times are very long ranging about 84-100 hours and the reaction never goes to completion.

Further the preparation of intermediate of the formula 5 from 3-hydroxy-4-methoxy-5-nitrobenzadehyde of the formula 6 as described in the scheme-1 has to be purified repeatedly and yield of the intermediate is only about 55%. Consequently the yield of the final product is also very low.

The critical raw material 3,4-dihydroxy-5-nitrobenzaldehyde of the formula 5 a catechol derivative, changes its colour from light yellow to dark colour on storage at room temperature in short period of time due to which the final product entacapone yield and quality is varies between the batches and there is no consistency in yield and quality. Hence storing of this compound required special conditions such as below 15°C under dark room.

In addition it was known in the literature that the catechol derivatives are known to under goes the aerial oxidation and gives quinone derivatives which contributes the colour changes over the storage.

Hence it is of paramount important to find out the stable penultimate stage intermediate which can be stable enough at room temperature for longer period and it can be stored and used as and when it is required and it was our target to improve the overall yield of the final product Entacapone which in the present route is about 58%.

Therefore the main objective of the present invention is to provide an improved process for the preparation of ENTACAPONE of the formula (1).

Another objective of the present invention is to provide an improved process for the preparation of ENTACAPONE of the formula (1) using 3-alkoxy-4-hydroxy-5-nitrobenzadehyde of the formula (2) which is a guaiacol derivative and the product of the formula (4) where R = methyl or ethyl, which is also a guaiacol derivative is stable at room temperature. It is not found to change the color for two months storage.

Yet another objective of the present invention is to provide an improved process for the preparation ENTACAPONE of the formula (1) by reducing the reaction time and making the reaction going to completion thereby making the process economical.

Still another objective of the present invention is to provide an improved process for the preparation of ENTACAPONE of the formula (1) by avoiding the use of 3,4-dihydroxy-5-nitrobenzaldehyde of the formula (5).

Yet another objective of the present-invention is to provide an improved process for the preparation of ENTACAPONE of the formula (1) employing intermediates of the formula 4 where R = methyl or ethyl.

Another objective of the present invention is to provide a novel intermediate of the formula 4 where R = ethyl.

Still another objective of the present invention is to provide a process for the preparation of intermediate of the formula 4 where R = methyl or ethyl.

The compound of the formula 4 wherein R = methyl is known as metabolite in Entacapone viz; Drug Metabolism and Disposition (1993), 21, 81-92 but has hitherto been prepared by any chemical method. Further the compounds of the formula 4 where R = methyl or ethyl have not been hitherto known as an intermediate for the preparation of Entacapone.

To achieve the above objectives, we devised an entirely different strategy for the preparation of the Entacapone of the formula (1).

Accordingly the process envisaged and developed by us involves the reaction shown in the scheme 2

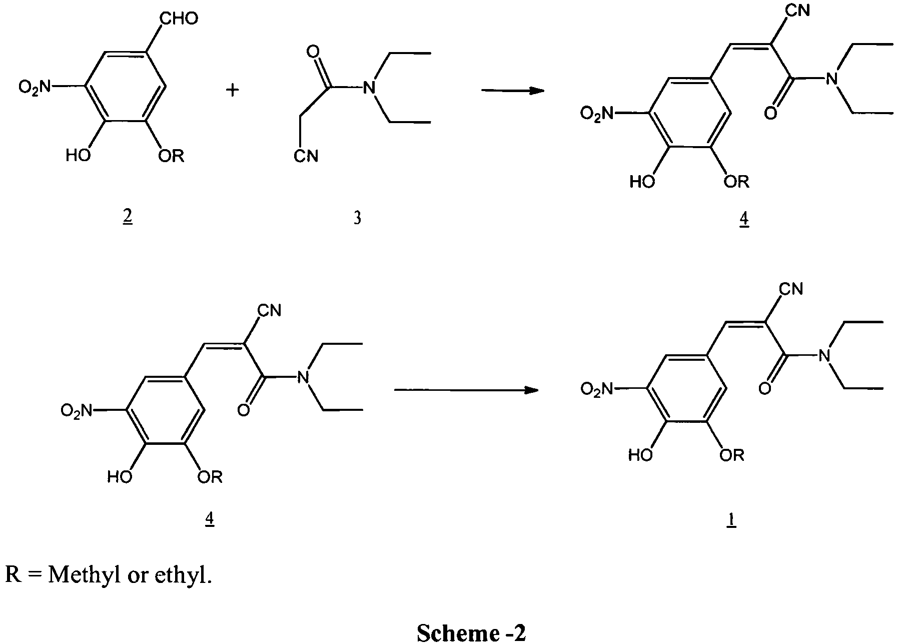

Accordingly, the present invention provides an improved process for the preparation of Entacapone of the formula (1) which comprises.
(i) reacting 3-alkoxy-4-hydroxy-5-nitrobenzadehyde of the formula (2) with N,N-diethylaminocyanoactamide of the formula (3) in the presence of mild acid catalyst and a solvent at a temperature in the range of 50-115°C, to get the intermediates of the formula (4) wherein R is methyl, ethyl.
(ii) Treating the 3-O-alkylated (methyl or ethyl) Entacapone of the formula (4) with acid catalyst in the presence of organic base and solvent at a temperature in the range of 20-60°C to get crude Entacapone of the formula (1).
(iii) purifying the crude entacapone obtained using solvent or a mixture thereof.

In a preferred embodiment of the invention the Step (i) may be carried out using solvents such as alcohols with carbon (C1 to C5) and toluene in presence of pyridine and piperidine salts such as peperidinium acetate, piperidinium propionate and pyridinium para toluene sulfonate etc. The reaction temperature used in this step can be preferably between 70-115°C and more preferably between 110 to 115°C. The solvent which can be employed is selected from isopropyl alcohol, ethanol, n-butanol, and toluene etc.

We have observed that the intermediate of the formula 4 where R = methyl or ethyl exist in two isomeric forms. Though we could separate the isomer in respect of the compound where R = methyl, we could not, in spite of our repeated and best efforts separate the isomers of the compound where R = ethyl.

However it is to be noted that the non separation of the isomers does not affect the conversion of the compound of the formula 4 where R is methyl or ethyl for its conversion to Entacapone of the formula (1).

Another significant aspect of this invention is that intermediate of the formula (4) where R = methyl or ethyl having a cyano group is very susceptible for hydrolysis even at mild acidic and basic condition it can convert to corresponding amide or acid and subsequently we may get the starting material of the formula (2) but by careful designing and execution of the dealkylation to get ENTACAPONE in good yield by doing the reaction at room temperature between 25-50°C.

Another aspect of this invention is that the reaction time for condensation of the formula (2) and formula (3) is only 17 hours and the reaction goes to completion thereby increasing the yield to 86%. The overall yield from compound of the formula (2) over two stages is 80%.

Yet another importance of this invention is that the intermediate of the formula (4) where R = methyl or ethyl is stable and there is no colour change occurred during the normal storage condition.

The another aspect of this invention is that the step (ii) is that the dealkylation which normally required higher temperature is presently carried out at of about 25-50°C. The solvents which can be employed is selected from chloroform, methylene dichloride and ethylene dichloride and tetahydrofuran etc.

The solvents which can be employed purification of the crude entacapone may be selected from like toluene, isopropylalcohol, methanol and acetone or their mixtures thereof.

The details of the process of this invention are described in the Examples given below which are provided only by way of illustration.

### EXAMPLE-1

### STEP-1: PREPARATION OF N,N-DIETHYL -2- CYANO -3- (-3-ETHOXY-4-HYDROXY-5 NITROPHENYL) ACRYLAMIDE OF THE FORMULA 4 WHERE R = ETHYL:

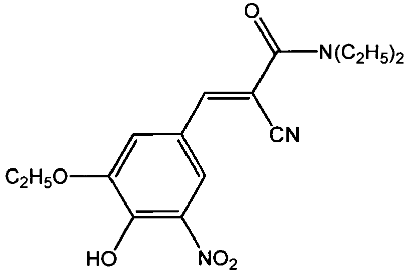

Charge 3-ethoxy-4-hydroxy-5-nitrobenzaldehyde 20g (0.0947mole) of the formula 2 where R = ethyl and N,N-diethylamino cyanoacetamide of the formula (3) 14.6g, (0.1042), acetic acid 3.13g and piperidine 4.45g along with toluene 200ml and heated to reflux temperature of about 100-115°C with continuous removal of water azeotrophically 15 hours. After the reaction is over the reaction mixture is concentrated to a volume of 20-30ml quenched in to dilute hydrochloric acid and chilled water 200ml and stirred for 60 minutes. The precipitated solid was filtered and dried to get 30g (95%), the HPLC purity is 94% (including isomer) of N,N-DIETHYL -2-CYANO-3-(-3-ETHOXY-4-HYDROXY-5-NITROPHENYL).

ACRYLAMIDE. The product is used as such directly to the next stage. MR = 110.1-117.2°C.

### STEP-2: PREPARATION OF N,N-DIETHYL-2-CYANO-3-(3,4-DIHYDROXY-5-NITROPHENYL)ACRYLAMIDE(ENTACAPONE)

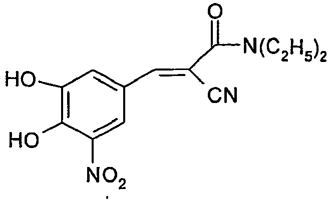

N,N-diethyl-2-cyano-3-(3-ethoxy-4-hydroxy-nitrophenyl) acrylamide 5g (0.0150 mole) of the formula 4 where, R= ethyl obtained by the process described in step(i) above is charged with pyridine 12.5ml along with dichloromethane 50 ml and stirred and cooled to 0-5° degrees and slowly charged the aluminium chloride 10g(0.751m) keeping the temperature below 5°and stirred at 0-5° for 30 minutes. After maintaining for 30 minutes the reaction mixture was slowly raised to 40-45°C and stirred at that temperature for 50 hours. After reaction completion the solvent is removed to get a residual volume of 10 ml and quenched in to dilute hydrochloric acid and chilled water (50ml) and stirred for 30 minutes. The formed product was filtered and dried. The dried weight is 4g (87.3%) ( with HPLC purity of about 93.25%. it was purified using methanol and toluene as a solvent to get the ENTACAPONE as a pure product having HPLC purity of 99.5% and the IR matching with the reported spectrum.
MR = 162-163°C
HPLC = 99.76%
PMR = 200MHz (DMSO-d6): δ value; 1.26 (m, 6H), 3.49( q, 4H), 7.50(s, 1H,), 7.905 (d, 1H,J=0.01), 7.99( 1H, J=0.01).
MS = M/Z = 306.4 (M+1),

### EXAMPLE-2

### STEP-1: PREPARATION OF N,N-DIETHYL -2-CYANO -3-( -3- ETHOXY -4-HYDROXY-5-NITROPHENYL) ACRYLAMIDEOF THE FORMULA 4 WHERE R = METHYL:

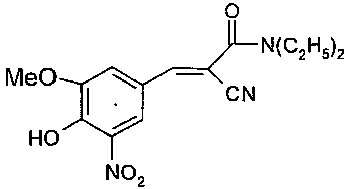

Charged 3-methoxy4-hydroxy-5-nitrobenzaldehyde 25g (0.126 mole) of the formula **2,** where R= methyl ) and N,N-diethylamino cyanoacetamide of the formula (3) ) 22.2g,( 0.158 mole) acetic acid 4.18g and piperidine 5.94g along with toluene 250ml and heated to reflux temperature of about 105-110° and remove the water azeotrophically for 15 hours. The reaction mixture is concentrated and quenched in to dilute hydrochloric acid and chilled water 375ml and stirred for 3 hours. The precipitated solid was filtered and dried to get 36g (88.95%) with HPLC purity is 94.2% (including isomer) and the N,N-DIETHYL -2-CYANO -3-( -3- METHOXY -4-HYDROXY-5-NITROPHENYL) ACRYLAMIDE which is used as such for the next stage.

Small sample was purified to get the pure single isomer (HPLC) by crystallising form methanol. The pure product is having the following properties.
HPLC PURITY : 99.63%
MR ; 130-132° C
IR: (Cm⁻¹): 2204 ( -CN), 1637 ( -C=O),
PMR( 200MHz), δ value; 1.28(m, 6H), 3.49 (q, 4H), 4.02(s, 3H), 7.608(s, 1H,), 8.02(d, 1H, J=0.01), 7.98( s, 1H, J=0.01),
MS= M/Z= 320.2 (M⁺¹)

### STEP-2 PREPARATION OF N,N- DIETHYL-2-CYANO-3-(3,4-DIHYDROXY-5-NITROPHENYL) ACRYLAMIDE (ENTACAPONE):

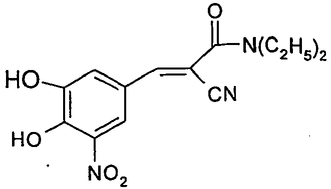

N,N-diethyl-2-cyano-3-(3-methoxy-4-hydroxy-5-nitrophenyl) acrylamide (20g) (0.062mole) of the formula **4** where , R=methyl obtained by the process described in step(i) of Example -2 is charged with pyridine 50ml along with dichloromethane 120 ml and stirred and cooled to 0-5 degrees and slowly charged the aluminium chloride 32g (0.239 mole) keeping the temperature below 5°and stirred at 0-5° C for 30 minutes. After maintaining for 30 minutes the reaction mixture was slowly raised to room temperature to 40-45°C and stirred for 2 hours. The reaction mixture quenched in dilute hydrochloric acid and ice water (50ml) after removing the methylene chloride and stirred for 60 minutes. The formed product was filtered and dried. The dried weight is 18g (94.1%) with HPLC purity of about 94.42%.

It was purified using methanol and toluene as a solvent to get the ENTACAPONE as a pure product having HPLC purity of 99.5%. it is matching with in all respect with the product obtained from the Example-1 **(step-2).**

### Advantages of the invention

The process is simple and economical as the reaction time reduced and the reaction goes to completion.

The process avoids the use of 3,4-dihydroxy-5-nitrobenzaldehyde of the formula (**5**)

The process results in a new intermediate of the formula 4 Wherein R= ethyl

## Claims

1. A process for the preparation of Entacapone of the formula (1) 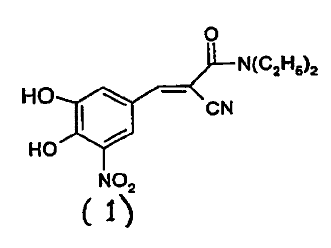 which comprises
(i) reacting 3-alkoxy-4-hydroxy-5-nitrobenzadehyde of the formula (2) with N,N-diethylaminocyanoactamide of the formula (3) in the presence of mild acid catalyst and a solvent at a temperature in the range of 50-115°C, to get the intermediates of the formula (4) wherein R is methyl or ethyl, 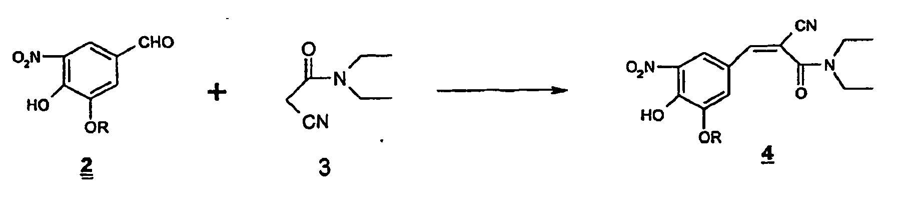
(ii) Treating the 3-O-alkylated (methyl or ethyl) entacapone of the formula (4) where R = methyl or ethyl with acid catalyst in the presence of organic base and solvent at a temperature in the range of 20-60°C to get crude entacapone of the formula (1) and if desired,
(iii) purifying the crude entacapone obtained using solvent or a mixture thereof.

2. A process as claimed in claim 1 wherein the solvent used in Step (i) is selected from an alcohol with carbon (C1 to C5) and toluene.

3. A process as claimed in claims 1 or 2 wherein the step (i) is carried out in the presence of a pyridine or piperidine salt.

4. A process as claimed in claim 3 wherein the pyridine or piperidine salt is piperidine acetate, piperidine propionate, pyridinium acetate, pyridium propionate or pyridinium para toluene sulfonate.

5. A process as claimed in any of claims 1 to 4 wherein the reaction temperature used in the step (i) ranges between 60-115degrees C.

6. A process as claimed in any of claims 1 to 5 wherein the solvent used in step (i) is selected from isopropyl alcohol, ethanol, n-butanol and toluene.

7. A process as claimed in any of claims 1 to 6 wherein the reaction time for the condensation of the formula (2) and formula (3) ranges between 15 to 25 hours.

8. A process as claimed in any of claims 1 to 7 wherein the reaction temperature for step (ii) ranges from 25-50 degrees C.

9. A process as claimed in any of claims 1 to 8 wherein the solvent used in step (ii) is selected from chloroform, methylene dichloride and ethylene dichloride and tetrahydrofuran.

10. A process as claimed in any of claims 1 to 9 wherein the solvent used for purification in step (iii) is selected from toluene, isopropylalcohol, methanol and acetone or their mixtures.

11. An intermediate of the formula (4) 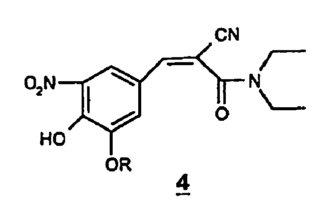 wherein R is ethyl.

12. A process for the preparation of intermediates of the formula (4) wherein R is methyl or ethyl which comprises reacting a compound of the formula (2) where R = methyl or ethyl with N, N-diethylaminocyanoactamide of the formula (3) in the presence of mild catalyst and a solvent at a temperature in the range of 50-115 degrees C, to get the intermediates of the formula (4) wherein R is methyl or ethyl

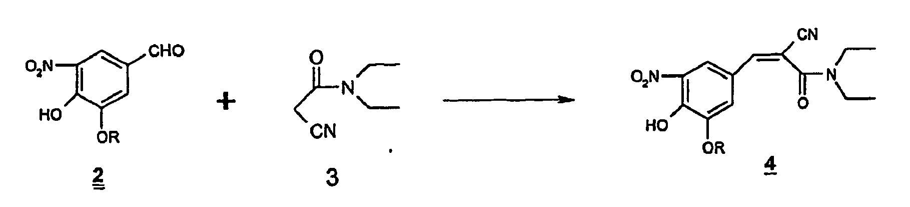

13. A process as claimed in claim 12 wherein the reaction temperature used in the process ranges between 60-115 degrees C.

14. A process as claimed in claim 12 or 13 wherein the solvent used is selected from an alcohol with carbon (C1 to C5) and toluene.

15. A process as claimed in any of claims 12 to 14 wherein the process is carried out in the presence of a pyridine or piperidine salt.

16. A process as claimed in claim 15 wherein the pyridine or piperidine salt is an acetate, propionate or para toluene sulfonate salt.

## Patentansprüche

1. Verfahren zur Herstellung von Entacapon der Formel (1) 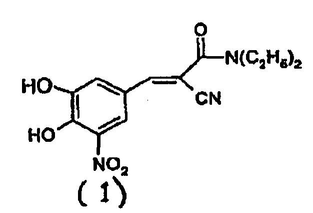 welches umfasst:
(i) Umsetzen von 3-Alkoxy-4-hydroxy-5-nitrobenzaldehyd der Formel (2) mit N,N-Diethylaminocyanacetamid der Formel (3) in der Gegenwart eines milden Säurekatalysators und eines Lösungsmittels bei einer Temperatur im Bereich von 50-115°C, um die Intermediate der Formel (4) zu erhalten, worin R Methyl oder Ethyl ist, 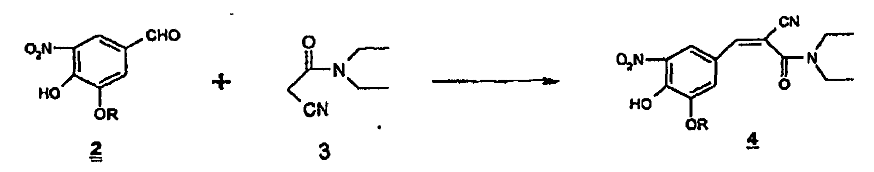
(ii) Behandeln des 3-O-alkylierten (Methyl oder Ethyl) Entacapons der Formel (4), worin R = Methyl oder Ethyl ist, mit Säurekatalysator in der Gegenwart von organischer Base und Lösungsmittel bei einer Temperatur im Bereich von 20-60°C, um rohes Entacapon der Formel (1) zu erhalten, und, sofern erwünscht,
(iii) Reinigen des erhaltenen rohen Entacapons unter Verwendung von Lösungsmittel oder eines Gemischs davon.

2. Verfahren, wie in Anspruch 1 beansprucht, wobei das in Schritt (i) verwendete Lösungsmittel ausgewählt ist aus einem Alkohol mit Kohlenstoffen C1 bis C5 und Toluol.

3. Verfahren, wie in Anspruch 1 oder 2 beansprucht, wobei der Schritt (i) in der Gegenwart eines Pyridin- oder Piperidinsalzes durchgeführt wird.

4. Verfahren, wie in Anspruch 3 beansprucht, worin das Pyridin- oder Piperidinsalz Piperidinacetat, Piperidinpropionat, Pyridinacetat, Pyridinpropionat oder Pyridinium-paratoluolsulfonat ist.

5. Verfahren, wie in einem der Ansprüche 1 bis 4 beansprucht, wobei die in Schritt (i) angewendete Reaktionstemperatur im Bereich zwischen 60-115°C liegt.

6. Verfahren, wie in einem der Ansprüche 1 bis 5 beansprucht, wobei das in Schritt (i) verwendete Lösungsmittel ausgewählt ist aus Isopropylalkohol, Ethanol, n-Butanol und Toluol.

7. Verfahren, wie in einem der Ansprüche 1 bis 6 beansprucht, wobei die Reaktionsdauer für die Kondensation der Formel (2) und Formel (3) im Bereich zwischen 15 und 25 Stunden liegt.

8. Verfahren, wie in einem der Ansprüche 1 bis 7 beansprucht, wobei die Reaktionstemperatur für Schritt (ii) im Bereich von 25-50°C liegt.

9. Verfahren, wie in einem der Ansprüche 1 bis 8 beansprucht, wobei das in Schritt (ii) verwendete Lösungsmittel ausgewählt ist aus Chloroform, Methylendichlorid und Ethylendichlorid, und Tetrahydrofuran.

10. Verfahren, wie in einem der Ansprüche 1 bis 9 beansprucht, wobei das für die Reinigung in Schritt (iii) verwendete Lösungsmittel ausgewählt ist aus Toluol, Isopropylalkohol, Methanol und Aceton oder aus deren Gemischen.

11. Intermediat der Formel (4) 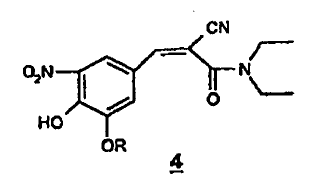 worin R Ethyl ist.

12. Verfahren für die Herstellung von Intermediaten der Formel (4), worin R Methyl oder Ethyl ist, welches das Umsetzen einer Verbindung der Formel (2) umfasst, worin R = Methyl oder Ethyl ist, mit N,N-Diethylaminocyanacetamid der Formel (3) in der Gegenwart eines milden Säurekatalysators und eines Lösungsmittels bei einer Temperatur im Bereich von 50-115°C, um die Intermediate der Formel (4) zu erhalten, worin R Methyl oder Ethyl ist,

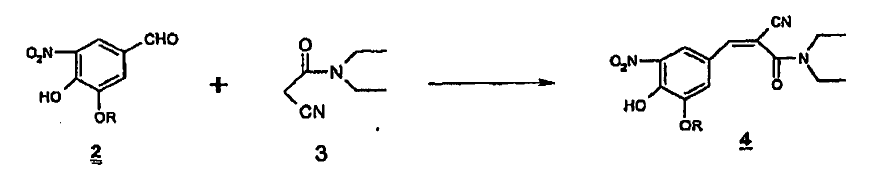

13. Verfahren, wie in Anspruch 12 beansprucht, wobei die im Verfahren angewendete Reaktionstemperatur im Bereich zwischen 60-115°C liegt.

14. Verfahren, wie in Anspruch 12 oder 13 beansprucht, wobei das verwendete Lösungsmittel ausgewählt ist aus einem Alkohol mit Kohlenstoffen C1 bis C5 und Toluol.

15. Verfahren, wie in einem der Ansprüche 12 bis 14 beansprucht, wobei das Verfahren in der Gegenwart eines Pyridin- oder Piperidinsalzes durchgeführt wird.

16. Verfahren, wie in Anspruch 15 beansprucht, wobei das Pyridin- oder Piperidinsalz ein Acetat-, Propionat- oder para-Toluolsulfonatsalz ist.

## Revendications

1. Procédé de préparation d'entacapone de formule (1) 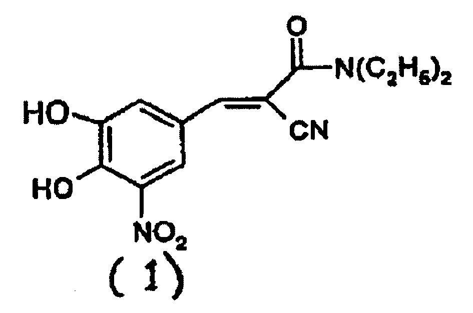 qui comprend
(i) la réaction du 3-alcoxy-4-hydroxy-5-nitrobenzaldéhyde de formule (2) avec du N,N-diéthylaminocyanoactamide de formule (3) en présence de catalyseur acide faible et un solvant à une température dans la plage de 50 à 115°C, pour obtenir les intermédiaires de formule (4) dans laquelle R est un groupe méthyle ou éthyle, 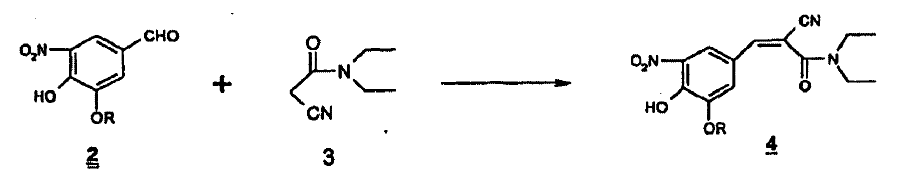
(ii) le traitement de l'entacapone 3-O-alkylée (méthyle ou éthyle) de formule (4) dans laquelle R = un groupe méthyle ou éthyle avec un catalyseur acide en présence d'une base organique et d'un solvant à une température dans la plage de 20 à 60°C pour obtenir une entacapone brute de formule (1) et si on le souhaite,
(iii) la purification de l'entacapone brute obtenue en utilisant le solvant ou un mélange de celui-ci.

2. Procédé selon la revendication 1, dans lequel le solvant utilisé à l'étape (i) est choisi parmi un alcool avec du carbone (en C1 à C5) et du toluène.

3. Procédé selon les revendications 1 ou 2, dans lequel l'étape (i) est réalisée en présence d'une pyridine ou d'un sel de pipéridine.

4. Procédé selon la revendication 3, dans lequel la pyridine ou un sel de pipéridine est l'acétate de pipéridine, le propionate de pipéridine, l'acétate de pyridinium, le propionate de pyridium ou le para-toluène-sulfonate de pyridinium.

5. Procédé selon l'une quelconque des revendications 1 à 4, dans lequel la température réactionnelle utilisée à l'étape (i) se situe entre 60 et 115 degrés C.

6. Procédé selon l'une quelconque des revendications 1 à 5, dans lequel le solvant utilisé à l'étape (i) est choisi parmi l'alcool isopropylique, l'éthanol, le n-butanol et le toluène.

7. Procédé selon l'une quelconque des revendications 1 à 6 dans lequel la durée réactionnelle pour la condensation de la formule (2) et de la formule (3) se situe entre 15 et 25 heures.

8. Procédé selon l'une quelconque des revendications 1 à 7, dans lequel la température réactionnelle de l'étape (ii) est de 25 à 50 degrés C.

9. Procédé selon l'une quelconque des revendications 1 à 8, dans lequel le solvant utilisé à l'étape (ii) est choisi parmi le chloroforme, le dichlorure de méthylène et le dichlorure d'éthylène et le tétrahydrofurane.

10. Procédé selon l'une quelconque des revendications 1 à 9, dans lequel le solvant utilisé pour la purification à l'étape (iii) est choisi parmi le toluène, l'alcool isopropylique, le méthanol et l'acétone ou leurs mélanges.

11. Intermédiaire de formule (4) 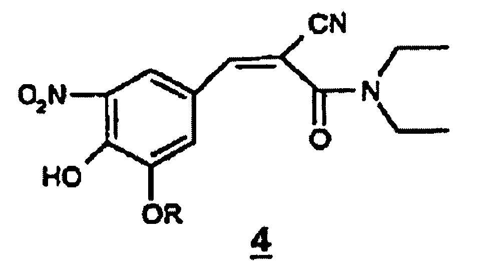 dans laquelle R est un groupe éthyle.

12. Procédé de préparation d'intermédiaires de formule (4) dans laquelle R est un groupe méthyle ou éthyle, qui comprend la réaction d'un composé de formule (2) dans laquelle R = un groupe méthyle ou éthyle avec le N,N-diéthylaminocyano-actamide de formule (3) en présence d'un catalyseur faible et un solvant à une température dans la plage de 50 à 115 degrés C, pour obtenir les intermédiaires de formule (4) dans laquelle R est un groupe méthyle ou éthyle

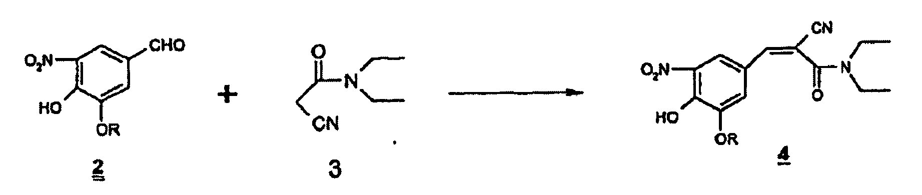

13. Procédé selon la revendication 12, dans lequel la température réactionnelle utilisée dans le procédé se situe entre 60 et 115 degrés C.

14. Procédé selon la revendication 12 ou 13, dans lequel le solvant utilisé est choisi parmi un alcool avec du carbone (en C1 à C5) et du toluène.

15. Procédé selon l'une quelconque des revendications 12 à 14, ledit procédé étant réalisé en présence d'une pyridine ou d'un sel de pipéridine

16. Procédé selon la revendication 15, dans lequel la pyridine ou le sel de pipéridine est un acétate, un propionate ou un sel de para-toluène sulfonate.
